# EUROPÄISCHE PATENTSCHRIFT

(11) **EP 1 163 281 B1**
(45) Veröffentlichungstag und Bekanntmachung des Hinweises auf die Patenterteilung: **05.01.2005**
(21) Anmeldenummer: 00912619.4
(22) Anmeldetag: 17.03.2000
(51) Int. Cl.: C08G 59/68, A61K 6/087, C08L 63/00, C08G 59/18

(54) **EPOXYVERBINDUNGEN ENTHALTENDE ZWEIKOMPONENTIGE ZUBEREITUNGEN**
TWO-COMPONENT PREPARATIONS CONTAINING EPOXY COMPOUNDS
COMPOSITIONS A DEUX COMPOSANTS CONTENANT DES COMPOSES EPOXY

(30) Priorität: 18.03.1999 DE 19912251
(43) Veröffentlichungstag der Anmeldung: 19.12.2001
(73) Patentinhaber: 3M ESPE AG, 82229 Seefeld (DE)
(72) Erfinder: ECKHARDT, Gunther, D-06231 Bad Dürrenberg (DE); GANGNUS, Bernd, D-82346 Andechs (DE); WEINMANN, Wolfgang, D-82205 Gilching (DE); FÜHRER, Cornelia, D-82211 Herrsching (DE)
(86) Internationale Anmeldenummer: PCT/EP2000/002388
(87) Internationale Veröffentlichungsnummer: WO 2000/056800

(56) Entgegenhaltungen:
- EP-A- 0 688 804
- EP-A- 0 764 691
- WO-A-96/13538
- DE-A- 4 324 322
- DE-A- 19 648 283
- DE-A- 19 753 461

## Beschreibung

Die Erfindung betrifft zweikomponentige, Epoxyverbindungen enthaltende Zubereitungen, besonders zur Herstellung von Dentalmassen. Insbesondere betrifft die Erfindung Epoxyverbindungen enthaltende zweikomponentige Dentalmassen, die durch kationische Polymerisation ausgehärtet werden.

Ein wichtiger Parameter bei mehrkomponentigen Dentalmassen ist deren Verarbeitungszeit. Hierunter wird die Zeit vom Abbindebeginn nach dem Vermischen der Komponenten bis zum Aushärten der Masse verstanden. Der Anwender benötigt nach dem Anmischen der Komponenten der Dentalmasse einen exakt definierten Zeitraum, in dem er die Masse problemlos handhaben kann. Direkt nach diesem Zeitraum soll die Masse in kürzester Zeit erhärten. Ein langsames Verstrammen der Masse während der Ver- bzw. Bearbeitung ist für den Anwender untragbar.

Aus dem Stand der Technik sind verschiedene Systeme bekannt, die den Abbindeverlauf einer aushärtenden Dentalmasse einzustellen versuchen.

Die DE-A-197 53 461 beschreibt beispielsweise lagerstabile kationisch polymerisierende Zubereitungen, bei denen lösliche und/oder feinteilige Erdalkaliund/oder Alkaliverbindungen eine Einstellung des Abbindeverlaufs erlauben. Das dort beschriebene Initiatorsystem kann im Falle von zweikomponentigen Formulierungen u.a. aus freien Lewis- bzw. Brönstedsäuren bestehen. Nachteilig an diesen Zubereitungen ist, dass sie nur einen sehr begrenzten Zeitraum für die Einstellung des Abbindeanfangs erlauben und zusätzlich bei einer Erhöhung der Konzentration der Erdalkali- bzw. Alkalimetalle zum Zwecke der Aufweitung des Abbindebereichs das Abbindeende stark verschleppen und die mechanischen Eigenschaften stark negativ beeinträchtigen.

Die DE-A-197 42 980 beschreibt zwar prinzipiell kationisch polymerisierbare Massen, jedoch auf auf der Basis von ROMP-Oligomeren bzw. -Polymeren, wobei die Massen mit epoxyfunktionellen Comonomeren versetzt sein können. In dem angegebenen Katalysatorsystem kommen freie Lewis- oder Brönstedsäuren zum Einsatz. Nachteilig an diesem System ist die Tatsache, dass der Abbindeverlauf nicht eingestellt werden kann. Nach Beginn der Polymerisation startet diese durch und führt in äußerst geringer Zeit zu einem harten Material mit geringem Volumenschrumpf.

Die DE-A-195 02 751 beschreibt photohärtbare Modellmaterialien für die Zahntechnik, bei denen durch eine geeignete Lichtquelle Lewis-Säuren gebildet werden können. Zwar besitzen photohärtbare Materialien prinzipiell eine beliebig lange Verarbeitungszeit, doch in der Praxis würde diese Eigenschaft nur im Dunkelraum zum Tragen kommen. Im Dentalbereich wird jedoch ungeschützt unter Tageslicht oder einer künstlichen starken Lichtquelle gearbeitet, die zu einer allmählichen Verstrammung des Dentalmaterials führen, womit diese Initiierungssysteme zur Herstellung von Dentalmaterialien mit breit einstellbarer Verarbeitungszeit nicht geeignet sind.

Weitere polymerisierbare Massen auf der Basis von Epoxiden mit Lewis- bzw. Brönstedsäuren in freier oder reaktiver Form als Katalysator- bzw. Initiatorsystem sind aus der DE-A-196 48 283 bekannt. Nachteilig an diesen Massen ist wiederum, dass sich die Abbindecharakteristik nicht einstellen lässt.

Der Erfindung liegt die Aufgabe zugrunde, Dentalmassen zur Verfügung zu stellen, die die Nachteile aus dem Stand der Technik nicht aufweisen.

Diese Aufgabe wird gelöst durch zweikomponentige Zubereitungen, umfassend die Komponenten (I) und (II), wobei mindestens eine Komponente Epoxyverbindungen enthält und die Zubereitung nach dem Mischen der beiden Komponenten durch kationische Polymerisation, initiiert durch Lewis- und/oder Brönstedsäuren, aushärtet, wobei die Lewis- und/oder Brönstedsäuren in Form von Vorläuferverbindungen enthalten sind, die zur Bildung von Lewis- und/oder Brönstedsäuren geeignet sind und bei oder nach dem Mischen der beiden Komponenten entweder durch chemische Umsetzung mittels Redoxreaktionen unter Verwendung von Bisaryliodoniumsalzen, Reduktionsmitteln und Kupferkomplexen in Verbindung mit Lewis- und/oder Brönstedsäuren in freier Form oder mittels durch Metallsalze unterstützter Dehalogenierung von Alkylhalogeniden gebildet werden.

Die Vorteile der aus den erfindungsgemäßen Zubereitungen hergestellten Dentalmassen liegen in der exakten Einstellbarkeit der Verarbeitungszeit sowie in den hervorragenden physikalischen Werten der ausgehärteten Dentalmassen.

Insbesondere wenn die Dentalmassen als Modellmaterialien zur Herstellung von Arbeitsmodellen in der Zahntechnik verwendet werden, zeigen sich weitere Vorteile: Verglichen mit Gips zeigen die erfindungsgemäßen Massen erhöhte mechanische Werte, wie Abrieb-, Zug- und Druckfestigkeit. Ebenso ist die Detailwiedergabe feiner Konturen und Rillen sowie die Dimensionstreue, die wesentlich durch einen möglichst geringen Polymerisationsschrumpf beeinflußt wird, erheblich besser. Verglichen mit bisher bekannten Modellmaterialen auf Kunststoffbasis, die sich durch eine eher umständliche Handhabung und/oder durch eine im Vergleich zu Gips zu lange Abbindephase auszeichnen, können die erfindungsgemäßen Dentalmassen automatisch angemischt und die Abbindephase hervorragend eingestellt werden. Das Ende der Abbindephase bestimmt den frühest möglichen Zeitpunkt, zu dem die Modelle entformt und weiterbearbeitet werden können.

Die Zubereitungen eignen sich ebenfalls für andere dentale Anwendungen, bei denen geringer Schrumpf von Vorteil ist, beispielsweise als Materialien zur Herstellung von provisorischen Kronen und Brücken sowie Befestigungszemente.

Die erfindungsgemäßen Zubereitungen umfasssen zwei Komponenten (I) und (II).

Die Epoxyverbindungen und die Lewis- und/oder Brönstedsäuren in Form von Verbindungen, die zur Bildung von Lewis- und/oder Brönstedsäuren befähigt sind und mit den Epoxyverbindungen nicht reagieren, können in beliebiger Verteilung in Komponente (I) und/oder in Komponente (II) vorliegen.

Aus den erfindungsgmäßen Zubereitungen erhaltene Dentalmassen enthalten, verteilt über die Komponenten (I) und (II), folgende Bestandteile:
(A) 10 bis 80 Gew.-%, vorzugsweise 10 bis 60 Gew.-%, Epoxyverbindungen,
(B) 0,01 bis 20 Gew.-%, vorzugsweise 0,1 bis 10 Gew.-%, Verbindungen, die zur Bildung von Lewis- oder Brönstedsäuren befähigt sind, und gegebenenfalls freie Lewis- und/oder Brönstedsäuren,
(C) 10 bis 89,99 Gew.-%, vorzugsweise 30 bis 89,99 Gew.-% Verdünnungsmittel,
(D) 0 bis 79,99 Gew.-%, vorzugsweise 15 bis 59,99 Gew.-%, Modifikatoren.

Epoxyverbindungen gemäß Bestandteil (A) können cycloaliphatische und/oder aromatische und/oder aliphatische Epoxyverbindungen mit mindestens zwei und/oder mindestens vier Epoxygruppen sein.

Cycloaliphatische Epoxide können beispielsweise die aus der DE-A-196 48 283 bekannten Epoxide sein, die nachfolgenden allgemeinen Formeln folgen: 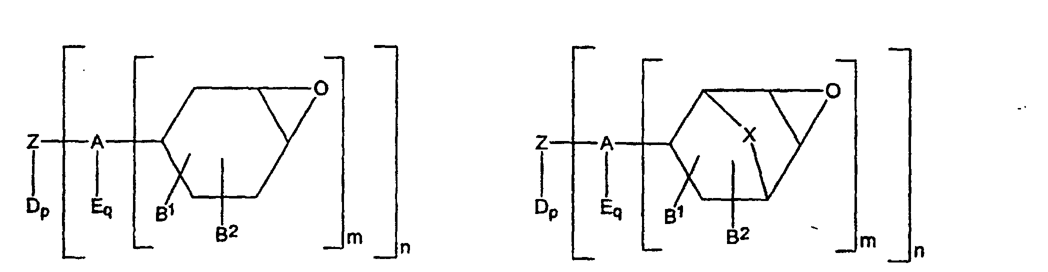 in welchen bedeuten:
- Z: einen aliphatischen, cycloaliphatischen oder aromatischen Rest mit 0 bis 22, vorzugsweise 0 bis 18 C-Atomen, oder eine Kombination dieser Reste, wobei ein oder mehrere C-Atome durch O, C=O, -O(C=O)-, SiR₂ und/oder NR ersetzt sein können, wobei R ein aliphatischer Rest mit 1 bis 7 C-Atomen ist, wobei ein oder mehrere C-Atome durch O, C=O und/oder -O(C=O)- ersetzt sein können,
- A: einen aliphatischen, cycloaliphatischen oder aromatischen Rest mit 1 bis 18, vorzugsweise 1 bis 15 C-Atomen oder eine Kombination dieser Reste, wobei ein oder mehrere C-Atome durch O, C=O, -O(C=O)-, SiR₂ und/oder NR ersetzt sein können, wobei R ein aliphatischer Rest mit 1 bis 7 C-Atomen ist, bei dem ein oder mehrere C-Atome durch O, C=O und/oder -O(C=O)- ersetzt sein können,
- B¹, B², D, E: unabhängig voneinander ein H-Atom oder einen aliphatischen Rest mit 1 bis 9, vorzugsweise 1 bis 7 C-Atomen, wobei ein oder mehrere C-Atome durch O, C=O, -O(C=O)-, SiR₂ und/oder NR ersetzt sein können, wobei R ein aliphatischer Rest mit 1 bis 7 C-Atomen ist, bei dem ein oder mehrere C-Atome durch O, C=O und/oder -O(C=O)- ersetzt sein können,
- X: CH₂, S oder O,
- n: 2 bis 7, vorzugsweise 2 bis 5,
- m: 1 bis 10, vorzugsweise 1 bis T,
- p: 1 bis 5, vorzugsweise 1 bis 4, und
- q: 1 bis 5, vorzugsweise 1 bis 4.

Es können auch niedrigviskose Epoxide, wie sie in der DE-A-196 48 283 beschrieben sind, eingesetzt werden.

Geeignet sind ebenfalls die aus der US-A-2 716 123, US-A-2 985 667, US-A-2 750 395, US-A-2 863 881, US-A-3 187 018, US-A-5 085 124, EP-A-0 449 027, EP-A-0 574 265 bekannten Epoxide, insbesondere Epoxide der folgenden Strukturformeln: 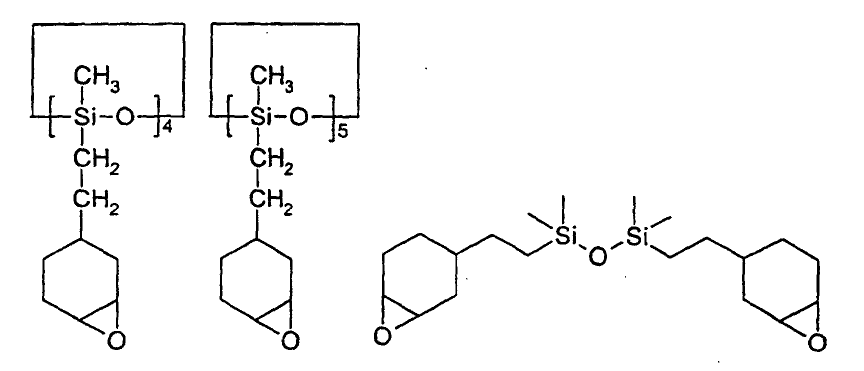 und

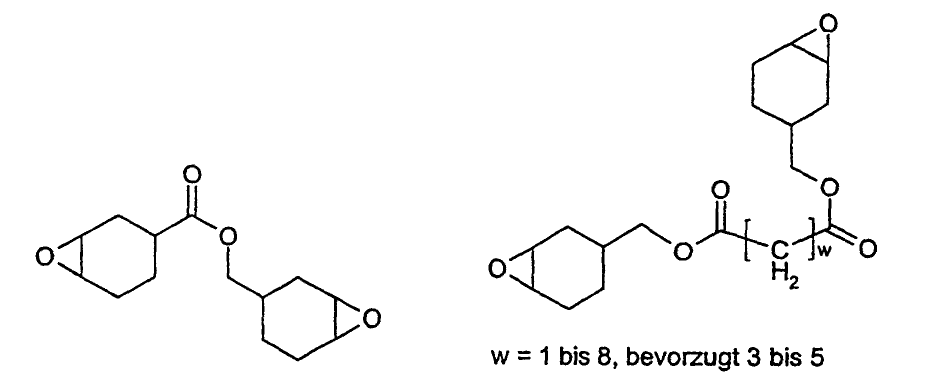

Kombinationen von aliphatischen, cycloaliphatischen oder aromatischen Epoxiden sind möglich. Bevorzugt werden cycloaliphatische Epoxyverbindungen mit mindestens zwei Epoxygruppen, cycloaliphatische Epoxyverbindungen mit mindestens vier Epoxygruppen oder die Kombination aus cycloaliphatischen Epoxyverbindungen mit zwei Epoxygruppen und cycloaliphatischen Epoxyverbindungen mit mindestens vier Epoxygruppen eingesetzt.

Die Lewis- und/oder Brönstedsäuren nach Bestandteil (B) werden durch chemische. Umsetzungen von ausgewählten Bestandteilen der beiden Komponenten bei oder nach deren Vermischung bilden. Freie Lewis- und/oder Brönstedsäuren, wie sie aus herkömmlichen Systemen bekannt sind, können ebenfalls eingesetzt werden.

Für den Fall, dass zusätzlich mit freien Lewis- oder Brönstedsäuren gearbeitet wird, hat es sich als zweckmäßig erwiesen, die Säuren derjenigen Komponente zuzusetzen, die keine Epoxyverbindungen enthält. Gegebenenfalls ist es zweckmäßig zur Einstellung der Verarbeitungszeit die aus der - zum Zeitpunkt dieser Anmeldung noch nicht-veröffentlichten - DE-A-197 53 461 bekannten Substanzen zur Verzögerung der kationischen Polymerisation ebenfalls einzusetzen.

Als Beispiel für freie Säuren seien genannt: BF₃ oder dessen Addukte, wie beispielsweise BF₃∃THF, BF₃∃Et₂O, AlCl₃, FeCl₃, HPF₆, HAsF₈, HSbF₈, HBF₄.

Die Brönsted- und/oder Lewissäuren werden durch ausgewählte Bestandteile, die in den beiden Komponenten getrennt gelagert werden und die bei oder nach der Vermischung der beiden Komponenten miteinander chemisch reagieren, erzeugt.

Reaktionen, die zu den für die Initiierung der Polymerisation von Epoxyverbindungen notwendigen Säuren führen können, sind Redoxreaktionen unter Verwendung von Bisaryliodoniumsalzen, Reduktionsmitteln und Kupferkomplexen und die durch Metallsalze unterstützte Dehalogenierung von Alkylhalogeniden.

Bei diesen latenten Initiierungssystemen ist es möglich, die jeweiligen Reaktionspartner sowohl den Epoxyverbindungen enthaltenden Komponenten als auch der keine Epoxyverbindungen enthaltenden Komponente zuzugeben.

Im allgemeinen ist die Aufteilung der Bestandteile der latenten Initiierungssysteme auf die beiden Komponenten so vorzunehmen, dass eine vorzeitige Polymerisation der Epoxyverbindungen bei der Lagerung der zweikomponentigen Materialien sicher vermieden wird.

Diese Aufgabenstellung wird beispielsweise durch Verwendung von nicht durch Säuren polymerisierbaren Komponenten erfüllt, die den besonders kritischen Bestandteil des jeweiligen latenten Initiierungssystems enthalten.

So hat es sich beispielsweise im Fall der Säurebildung durch Redoxreaktionen aus Bisaryliodoniumsalzen, Reduktionsmitteln und Kupferverbindungen als zweckmäßig erwiesen, die Bisaryliodoniumsalze und die Reduktionsmittel in einer keine Epoxyverbindungen enthaltenden Komponente zu lagern. Die Kupferverbindungen sind dann zusammen mit den Epoxyverbindungen in einer weiteren Komponente enthalten.

Die lodoniumsalze liegen zu 0,01 bis 20 Gew.-%, bevorzugt 0,2 bis 10 Gew.-%, bezogen auf die Masse des angemischten Materials in den Komponenten vor, die ebenfalls notwendigen Reduktionsmittel zu 0,01 bis 10 Gew.-%, bevorzugt 0,05 bis 5 Gew.-%, bezogen auf die Masse des angemischten Materials und die Kupferverbindungen zu 0,005 bis 10 Gew.-%, bevorzugt 0.01 bis 5 Gew.-%, bezogen auf die Masse des angemischten Materials.

Geeignet sind beispielsweise die Bisaryliodoniumverbindungen, die aus der US-A-4 225 691 und US-A-4 238 587 bekannt sind. Methoden zur Herstellung weiterer Diaryliodoniumverbindungen sind aus F.M. Beringer, R.A. Falk, M. Karmal, J. Lillien, G. Masullo, M. Mausner, E. Sommers, J. Am. Chem. Soc., 81, 342 (1958) und I. Mason, Nature, 139, 150 (1937) bekannt.

Besonders bevorzugt sind Diaryliodoniumverbindungen, die u.a. aus der DE-A-197 36 471 bekannt sind. Sie weisen folgende Struktur auf:

[((R¹)ₐAr¹)-I-(Ar²(R²)_{b})]⁺ Y⁻

Ar¹ und Ar² können unabhängig voneinander verschiedene, substituierte oder unsubstituierte, kondensierte oder nichtkondensierte aromatische Systeme mit 4 bis 20 C-Atomen sein, wie beispielsweise Phenyl, Tolyl, Cumyl, Anisyl, Chlorphenyl, Nitrophenyl, Naphthyl, Thienyl, Furanyl und Pyrazolyl, wobei R¹ und R² gleich oder verschieden sind und unabhängig voneinander ein H-Atom, einen aliphatischen Rest mit 1 bis 19, vorzugsweise 1 bis 9 C-Atomen, wobei ein oder mehrere C-Atome durch O, C=O, -O(C=O)-, F, Cl, Br, SiR³₃ und/oder NR³₂ ersetzt sein können, wobei R³ ein aliphatischer Rest mit 1 bis 7 C-Atomen ist, bei dem ein oder mehrere C-Atome durch O, C=O und/oder -O(C=O)- ersetzt sein können, bedeuten und a und b unabhängig voneinander 1 bis 5 sein können. Die Aromaten Ar¹ und Ar² können über R¹ und/oder R² miteinander verbunden sein.

Das Gegenanion Y⁻ ist ein wenig nucleophiles Anion der folgenden Struktur

KₓL_{y}⁻

wobei K ein Element der III., V. oder VII. Hauptgruppe, wie B, Al, P, Sb, As oder I ist und x Zahlenwerte von 1 bis 4 annehmen kann. L bedeutet unabhängig voneinander aromatische, aliphatische, araliphatische oder cycloaliphatische Reste mit 1 bis 25 C-Atomen, bei dem ein oder mehrere C-Atome mit F, Cl, Br oder I substituiert sein können und y Zahlenwerte von 0 bis 6 annehmen kann.

Bevorzugte Reste L sind Pentafluorophenyl-, Tetrafluorophenyl-, Trifluorophenyl, Fluorophenyl- Phenyl, 4-Trifluoromethylphenyl-, 3,5-Bis(trifluoromethyl)phenyl-, 2,4,6-Tris(trifluoromethyl)phenyl-, Fluor und lod.

Besonders bevorzugte Gegenanionen Y⁻ sind PF₈⁻, AsF₆⁻, SbF₆⁻, B(C₆F₅)₄⁻ und BF₄⁻.

Weitere Diaryliodoniumverbindungen sind beispielsweise auch in US-A-4 246 703 beschrieben.

Besonders geeignete Diaryliodoniumverbindungen sind:
- Diphenyliodoniumtetrafluoroborat
- Diphenyliodoniumhexafluorophosphat
- Diphenyliodoniumhexafluoroantimonat
- Diphenyliodoniumtetrakis(pentafluorophenyl)borat
- Bis-(4-methylphenyl)iodoniumhexafluorophosphat
- Bis-(4-methylphenyl)iodoniumhexafluoroantimonat
- Bis-(4-methylphenyl)iodoniumtetrakis(pentafluorophenyl)borat
- Phenyl-4-methylphenyliodoniumhexafluorophosphat
- Phenyl-4-methylphenyliodoniumhexafluoroantimonat
- Phenyl-4-methylphenyliodoniumtetrakis(pentafluorophenyl)borat
- Phenyl-4-methoxyphenyliodoniumhexafluoroantimonat
- Phenyl-4-methoxyphenyliodoniumtetrakis(pentaftuorophenyl)borat
- Phenyl-3-nitrophenyliodoniumhexafluorophenylantimonat
- Phenyl-3-nitrophenyliodoniumtetrakis(pentafluorophenyl)borat
- Bis(4-tert-butylphenyl)iodoniumhexafluoroantimonat
- Bis(4-tert-butylphenyl)iodoniumtetrakis(pentafluorophenyl)borat
- Phenyl 4-diphenyliodoniumhexafluoroantimonat
- Dinaphthyliodoniumhexafluorophosphat
- Dinaphthyliodoniumhexafluoroantimonat
- Dinaphthyliodoniumtetrakis(pentafluorophenyl)borat
- Bis(4-dodecylphenyl)iodoniumhexafluoroantimonat
- Bis(4-dodecylphenyl)iodonium tetrakis(pentafluorophenyl)borat
- 4-Methylphenyl-4-isopropylphenyliodoniumhexafluoroantimonat
- 4-Methylphenyl-4-isopropylphenyliodoniumtetrakis(pentafluorophenyl)borat

Reduktionsmittel zur Durchführung der Redoxreaktion zur Säurebildung sind organische oder anorganische Verbindungen oder Polymere, die befähigt sind, die Ladung des Heteroatoms innerhalb des Diaryliodoniumsalzes zu erniedrigen. Erwähnenswert sind Ascorbinsäure und deren Derivate, besonders das Palmitat, Oleat und Acetat Es können auch Zinn(II)-Verbindungen eingesetzt werden, beispielsweise Sn²⁺-Carboxylate, insbesondere Zinnoctoat, Zinnstearat, Zinnlaurat, Zinncitrat, Zinnoxalat, Zinnbenzoat. Unter den organischen Verbindungen sind α-Hydroxyverbindungen, beispielsweise Ketone, besonders Acyloine und Benzoine, zu nennen. Mit eingeschlossen sind auch Eisen(II)-Verbindungen, beispielsweise Ferrocene, FeBr₂, FeCl₂, reduzierende Zucker, wie Glucose, Fructose und Galactose, Phenole, wie Thiophenol, Silane und Organosiloxane.

Unter Kupferverbindungen sind Kupfer(I)- und Kupfer(II)-Verbindungen, beispielsweise Salze von Carbonsäuren und Mineralsäuren, wie CuBr, CuCl, Cu(II)-benzoat, Cu(II)-citrat, Cu(II)-formiat, Cu(II)-acetat, Cu(II)-stearat, Cu(II)-oleat, Cu(II)-carbonat, Cu(II)-gluconat, Cu(II)-naphthenoat oder Cu(II)-acetylacetonat zu verstehen.

Es sind auch Kupferchelate, wie sie in Cotton and Wilkinson, Advanced Inorganic Chemistry, 3rd Edition, Interscience Publishers, New York, 1972, Seiten 905 bis 922 erwähnt sind, verwendbar. Bevorzugte Kupferchelate sind solche, die in genügender Menge im kationisch polymerisierbaren Material inkorporiert oder dispergiert werden können. Besonders geeignet sind Kupferacetylacetonat, Kupfersalicylat, Cul(C₆H₅)₃P, Cul(C₂H₅O)₃P, CuCl₂C₂H₈N₂, [(N-C₄H₉)₄N]₂CUCl₄, etc.

Für die durch Metallsalze unterstützte Dehalogenierung von Alkylhalogeniden geeignete Alkylhalogenide werden in "Makromol. Chem." 178, 2139-2140 (1977) und in "Die Makromolekulare Chemie" 156 (1972) 325-328 beschrieben.

Besonders Verbindungen des Typs R₃C-X sind geeignet, worin X ein Halogen, bevorzugt Cl und Br, und R einen beliebigen substituierten oder unsubstituierten aliphatischen oder cycloaliphatischen Rest mit einer Kettenlänge von 1 bis 15 C-Atomen, bevorzugt 1 bis 8 C-Atomen, oder einen beliebig substituierten aromatischen Rest, darstellt. Es können auch Verbindungen mit bis zu drei verschiedene Substituenten am zentralen C-Atom verwendet werden.

Unter Metallsalzen sind anorganische oder organische Metallsalze zu verstehen, die befähigt sind, das Halogen aus den Verbindungen o.g. Typs zu abstrahieren. Möglich sind Salze wie AgBF₄, AgSbF₆, AlCl₃, ZnCl₂, aber auch BF₃. Allgemein geeignet sind lewissaure Verbindungen des Typus M(III)X₃, M(II)X₂, M(V)X₅ und deren mögliche Addukte mit AgX. X stellt hierbei ein Halogen dar, bevorzugt F, Cl oder Br und M ein Metall aus der betreffenden Haupt- oder Nebengruppe, bevorzugt aus der III. und V. Hauptgruppe, darin bevorzugt B, Al, Sb, As, und aus der I., II., IVa. und VIIIa. Nebengruppe, darin bevorzugt Cu, Ag, Zn, Fe, Co, Ni und Ti.

In mindestens einer der beiden Komponenten der erfindungsgemäßen Zubereitungen sind als Bestandteil (C) Verdünnungsmittel als Fließverbesserer vorhanden. Mit Vorteil sind Verdünnungsmittel einsetzbar, die üblicherweise als Weichmacher verwendet werden.

Typische Vertreter sind die Ester der Phthalsäure, wie Di-2-ethylhexylphthalat, oder die Ester von mehrbasischen aliphatischen Säuren, wie Dioctyladipat oder Acetyltributylcitrat.

Gut geeignet sind außerdem aliphatische und aromatische Kohlenwasserstoffe mit 6 bis 30 C-Atomen, die verzweigt oder unverzweigt sein können. Typische Beispiele sind Polypropylenöle oder Polyisobutylenöle. Mit Vorteil werden aromatische Kohlenwasserstoffe, wie Polyphenylenverbindungen, Dibenzyltoluol und Dibenzylphenylmethan, eingesetzt.

Es sind auch Polyesterpolyole einsetzbar, die beispielsweise durch Polykondensation aus niedermolekularen Polyolen und Polycarbonsäuren und/oder deren Anhydriden hergestellt werden können.

Typische Vertreter dieser Klasse werden von der Firma Hüls unter der Bezeichnung Dynacoll vetrieben. Bevorzugt werden solche Polyesterpolyole verwendet, die Molmassen zwischen 1000 und 5000 g/Mol und Hydroxyläquivalentmassen von 500 bis 2000 besitzen.

Besonders bevorzugt können solche Polyesterpolyole eingesetzt werden, wie sie durch katalysierte Reaktionen von Caprolacton mit unterschiedlichen Starteralkoholen zugänglich sind.

Typische Vertreter dieser Verbindungsklasse werden von der Firma UCC unter der Bezeichnung Tone oder von der Firma Daicel unter der Bezeichnung Placcel vertrieben.

Es können Polycaprolactontriole mit Molmassen von 200 bis 1000 g/Mol und Polycaprolactondiole mit Molmassen von 300 bis 2000 g/Mol verwendet werden.

Ferner können als Verdünnungsmittel Polycarbonatdiole mit Molmassen von 400 bis 2000 g/Mol und der allgemeinen Struktur 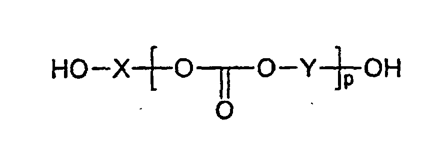 eingesetzt werden, wobei X und Y gleich oder verschieden sein können und unabhängig voneinander Alkylen-, Arylen-, Alkarylen-, Polyoxyalkylen- bedeuten und p Werte zwischen 1 und 50 annehmen kann.

Des weiteren können ggf. partiell epoxidierte Polybutandiole verwendet werden, die Homopolymerisate des Butadiens darstellen, die mit OH-Gruppen terminiert sind, Molmassen von 1000 bis 5000 g/Mol aufweisen und einen hohen Gehalt an Doppelbindungen besitzen, die ggf. durch Epoxidierung partiell zu mittelständigen aliphatischen Epoxidgruppen umgesetzt wurden. Vertreter dieser Verbindungsklasse werden von der Firma Atochem unter der Bezeichnung "Poly bd" vertrieben.

Schließlich sind auch Polyetherpolyole der allgemeinen Struktur: 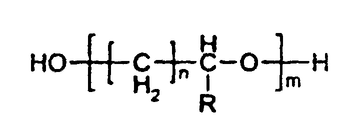 einsetzbar, wobei m eine ganze Zahl von 20 bis 150, n eine ganze Zahl von 1 bis 5 und R Wasserstoff oder C1 - C4-Alkyl bedeuten.

Bevorzugt werden Mischpolyetherpolyole, die aus Propylenoxid-Einheiten und/oder Ethylenoxideinheiten und/oder Tetrahydrofuraneinheiten bestehen, verwendet.

Mit Vorteil sind auch alkox-verlängerte Polyole, wie beispielsweise ethoxyliertes Bisphenol A oder propoxyliertes Trimethylolpropan einsetzbar.

Für die Einstellung der Eigenschaften kann es zweckmäßig sein, Mischungen von Polyolen unterschiedlicher mittlerer Molmasse und unterschiedlicher Struktur einzusetzen.

Unter Modifikatoren sind beispielsweise Füllstoffe zu verstehen. Füllstoffe können die im Dentalbereich üblichen, beispielsweise Quarz, Quarzmehl, gemahlene oder reaktive Gläser, Splitterpolymerisate, Kieselgele sowie pyrogene Kieselsäure oder deren Granulate, sein. Aber auch andere Füllstoffe wie beispielsweise feinverteilte Metall- oder Kunststoffpulver, Bariumsulfat, Titandioxid oder allgemein feinvermahltene Mineralien, sind geeignet. Zum besseren Einbau in die Polymermatrix kann es von Vorteil sein, die Füllstoffe zu hydrophobieren. Übliche Hydrophobierungsmittel sind Silane, beispielsweise Glycidyloxypropyltrimethoxysilan. Die maximale Komgröße der anorganischen Füllstoffe beträgt 100 µm, vorzugsweise 20 µm.

Als weitere Modifikatoren, wie Farbstoffe oder Thixotropiemittel, können die auf dem zahntechnischen Gebiet üblichen Stoffe eingesetzt werden.

Die beiden Komponenten der erfindungsgemäßen Zubereitungen können getrennt, beispielsweise in Doppelkammerkartuschen, aufbewahrt und vor Gebrauch durch Ausbringen über einen statischen oder dynamischen Mischer innig vermischt werden. Auch Handanmischvarianten sind möglich.

Das Verhältnis zwischen der keine Epoxyverbindungen enthaltenden Komponente und der Epoxyverbindungen enthaltenden Komponente kann 1 : 10 bis 1 : 1 und vorzugsweise 1 : 5 bis 1 : 2 betragen.

### Beispiele

Die Erfindung wird nachfolgend durch Beispiele aus dem Modellmaterialsektor näher beschrieben, die den Umfang der Erfindung in keinem Fall einschränken sollen. Die im Laufe dieser Beispiele verwendeten Substanzen sind von folgenden Herstellern zu beziehen:

| | |
|---|---|
| Aerosil R 805 | Degussa |
| Aerosil R202 | Degussa |
| Celloxide 2021 P | UCB |
| Citrofol B1 | Jungbunzlauer |
| Dianol 265 | Akzo Nobel |
| Jarytherm DBT | Elf Atochem |
| Placcel 305 | Interorgana(Daiccel) |
| Rhodorsil 2074 | Rhone-Poulenc |
| Silbond FW 100 EST | Quarzwerke Frechen |
| Silbond FW 300 EST | Quarzwerke Frechen |
| Silbond FW 600 EST | Quarzwerke Frechen |
| Titandioxid P 25 | Degussa |

Die Substanz CEPK ist ein cycloaliphatisches Epoxidharz mit vier Epoxygruppen, hergestellt nach Vorschriften der DE-A-196 48 283.

Die als Stabilisator verwendete Substanz LM wurde gemäß der DE-A-197 53 461, Beispiel 1 hergestellt.

Die Herstellung der in den folgenden Beispielen anhand ihrer Zusammensetzungen charakterisierten Basis-Pasten und Katalysator-Pasten erfolgte in 3-Finger-Knetern im 100g-Maßstab. Nach Erreichung eines homogenen Mischzustandes wurden die Pasten evakuiert und anschließend in 4:1-Doppelkammerkartuschen abgefüllt.

| Vergleichsbeispiel 1 | | | |
|---|---|---|---|
| Basis-Paste | | Katalysator-Paste | |
| Celloxide 2021 P | 49,899 % | Dianol 265 | 77,00 % |
| Silbond FW 600 EST | 49,000 % | Silbond FW 300 EST | 20,00 % |
| Aerosil R202 | 1,000 % | Aerosil R202 | 1,00 % |
| LM | 0,101 % | Titandioxid P 25 | 1,00 % |
| | | HSbF₆ 60%ig | 1,00% |

| Beispiel 1 | | | |
|---|---|---|---|
| Basis-Paste | | Katalysator-Paste | |
| Silbond FW 100 EST | 46,979 % | Silbond FW 100 EST | 46,944 % |
| CEPK | 16,992 % | Dianol 265 | 19,477 % |
| Silbond FW 600 EST. | 15,993 % | Silbond FW 600 EST | 15,981 % |
| Celloxide 2021 P | 8,996 % | Butyllactat | 8,490 % |
| Poly-THF 250 | 4,498 % | Rhodorsil 2074 | 5,528 % |
| Dianol 265 | 3,998 % | Aerosil R805 | 1,498 % |
| Aerosil R805 | 1,500% | Ascorbinsäure | 0,984 % |
| Kupfemaphtenoat 8%ig in Testbenzin | 0,544 % | HSbF₆ 60%ig | 0,599 % |
| Titandioxid P25 | 0,500 % | Titandioxid P 25 | 0,499% |

| Beispiel 2 (nicht erfindungsgemäß) | | | |
|---|---|---|---|
| Basis-Paste | | Katalysator-Paste | |
| Silbond FW 100 EST | 31,750 % | Silbond FW 100 EST | 62,500% |
| Silbond FW 600 EST | 31,750 % | Dianol 265 | 13,065 % |
| CEPK | 17,300% | Butyllactat | 8,500 % |
| Celloxide 2021 P | 10,315 % | Poly-THF 250 | 6,915 % |
| Dianol 265 | 4,570% | Rhodorsil 2074 | 5,535 % |
| Poly-THF 250 | 2,418 % | Aerosil R805 | 1,500 % |
| Aerosil R805 | 1,500 % | Titandioxid P 25 | 1,000 % |
| Kupferdimethacrylat | 0,397 % | Ascorbinsäure | 0,985 % |

| Beispiel 3 | | | |
|---|---|---|---|
| Basis-Paste | | Katalysator-Paste | |
| Silbond FW 100 EST | 47,845 % | Silbond FW 100 EST | 43,713 % |
| Silbond FW 600 EST | 14,952 % | Placcel 305 | 31,009 % |
| CEPK | 12,460 % | Silbond FW 600 EST | 15,005 % |
| Celloxide 2021 P | 12,460 % | Kupfertetrafluoroborat | 4,272 % |
| Placcel 305 | 4,485 % | Jarytherm DBT | 4,001 % |
| Jarytherm DBT | 3,987 % | Aerosil R805 | 1.500% |
| Diphenylmethylchlorid | 1,818 % | Titandioxid P 25 | 0,500 % |
| Aerosil R805 | 1,495 % | | |
| Titandioxid P 25 | 0,498 % | | |

| Beispiel 4 | | | |
|---|---|---|---|
| Basis-Paste | | Katalysator-Paste | |
| Silbond FW 100 EST | 47,025 % | Silbond FW 100 EST | 41,943 % |
| Silbond FW 600 EST | 14,976% | Dianol 265 | 18.475% |
| CEPK | 10,483 % | Silbond FW 600 EST | 14,980 % |
| Celloxide 2021 P | 10,483 % | Placcel 305 | 10,985 % |
| Dianol 265 | 6,490 % | Eisen-(II)-tetrafluoroborat | 6,068 % |
| Citrofol B1 | 5,990 % | Citrofol B1 | 4,993 % |
| Triphenylmethylchlorid | 2,556 % | Aerosil R805 | 1,498 % |
| Aerosil R805 | 1,498 % | Tetrafluorborsäure 48%ig | 0,559 % |
| Titandioxid P 25 | 0,499 % | Titandioxid P 25 | 0,499 % |

| Beispiel 5 | | | |
|---|---|---|---|
| Basis-Paste | | Katalysator-Paste | |
| Silbond FW 100 EST | 45,804 % | Silbond FW 100 EST | 44,432 % |
| CEPK | 16,589 % | Placcel 305 | 26,991 % |
| Silbond FW 600 EST | 14,990 % | Silbond FW 600 EST | 14,996 % |
| Celloxide 2021 P | 7,993 % | Jarytherm DBT | 7,997 % |
| Jarytherm DBT | 5,996 % | Zinktetrafluoroborat | 3,584% |
| Placcel 305 | 4,497 % | Aerosil R805 | 1,500 % |
| Triphenylmethylchlorid | 2,132 % | Titandioxid P 25 | 0,500 % |
| Aerosil R805 | 1,499 % | | |
| Titandioxid P 25 | 0,500 % | | |

Aus der Gegenüberstellung der Eigenschaften der erfindungsgemäßen Materialien mit handelsüblichen Produkten in Tabelle 1 geht klar hervor, dass die Materialien dem Zahntechniker Vorteile bieten.

| Material | Fräsbar nach [min] | Biegefestigkeit [Mpa] | Automatisch mischbar |
|---|---|---|---|
| Vergleichsbeispiel 1 | 30 | 105 ±14 | Ja |
| Beispiel 1 | 45 | 65±6 | Ja |
| Beispiel 2 | 45 | 99±7 - | Ja |
| Beispiel 3 | 30 | 62±5 | Ja |
| Beispiel 4 | 45 | 34 ± 3 | Ja |
| Beispiel 5 | 45 | 41 ± 1 | Ja |
| Vergleichsbeispiel 2 | 30-60 | 96 ± 2 | Nein |
| Vergleichsbeispiel 3 | 360-480 | 43 ± 2 | Nein |

Bei dem Vergleichsbeispiel 1, bei welchem nur freie Säure als Initiator eingesetzt worden war, war die Verarbeitungszeit mit 60 Sekunden zu kurz. Als Vergleichsbeispiel 2 dient ein Superhartgips der Marke Fujirock (GC ) und als Vergleichsbeispiel 3 ein konventionell härtendes Epoxyharz der Marke Blue Star Typ E (Girrbach).

## Patentansprüche

1. Zweikomponentige Zubereitung, umfassend die Komponenten (I) und (II), wobei mindestens eine Komponente Epoxyverbindungen enthält und die Zubereitung nach dem Mischen der beiden Komponenten durch kationische Polymerisation, initiiert durch Lewis- und/oder Brönstedsäuren, aushärtet, wobei die Lewis- und/oder Brönstedsäuren in Form von Vorläuferverbindungen enthalten sind, die zur Bildung von Lewis- und/oder Brönstedsäuren geeignet sind und bei oder nach dem Mischen der beiden Komponenten entweder durch chemische Umsetzung mittels Redoxreaktionen unter Verwendung von Bisaryliodoniumsalzen, Reduktionsmitteln und Kupferkomplexen in Verbindung mit Lewis- und/oder Brönstedsäuren in freier Form oder mittels durch Metallsalze unterstützter Dehalogenierung von Alkylhalogeniden gebildet werden.

2. Zubereitung nach Anspruch 1, **dadurch gekennzeichnet, dass** die Epoxyverbindungen und die zur Bildung von Lewis- und/oder Brönstedsäuren befähigten Verbindungen, die mit den Epoxyverbindungen nicht reagieren, in beliebiger Verteilung in Komponente (I) und/oder in Komponente (II) vorliegen.

3. Zubereitung nach Anspruch 1, **dadurch gekennzeichnet, daß** sie im Falle einer chemischen Umsetzung mittels durch Metallsalze unterstützter Dehlaogeniereung von Alkylhalogeniden zusätzlich als Teil derjenigen Komponente, die keine Epoxyverbindungen aufweist, Lewis- und/oder Brönstedsäuren in freier Form enthält.

4. Zubereitung nach Anspruch 3, **dadurch gekennzeichnet, dass** die Epoxyverbindungen in Komponente (I), die Lewis- und/oder Brönstedsäuren in freier Form in Komponente (II) und die Verbindungen, die zur Bildung von Lewis- und/oder Brönstedsäuren geeignet sind, die mit den Epoxyverbindungen nicht reagieren, in beliebiger Verteilung in Komponente (I) und/oder (II) vorliegen.

5. Dentalmasse aus Zubereitungen nach einem der Ansprüche 1 bis 4, **dadurch gekennzeichnet, dass** sie, verteilt über die Komponenten (I) und (II), die Bestandteile:
(A) 10 bis 80 Gew.-% Epoxyverbindungen,
(B) 0,01 bis 20 Gew.-% Verbindungen, die zur Bildung von Lewis- und/oder Brönstedsäuren befähigt sind, und gegebenenfalls freie Lewis- und/oder Brönstedsäuren,
(C) 10 bis 89,99 Gew.-% Verdünnungsmittel,
(D) 0 bis 79,99 Gew.-% Modifikatoren,
enthalten, wobei sich die Angabe der Gew.-% auf die Gesamtmasse der angemischten Zubereitungen bezieht.

6. Dentalmasse nach Anspruch 5, **dadurch gekennzeichnet, dass** als Bestandteil (A) cycloaliphatische Epoxyverbindungen und/oder aromatische Epoxyverbindungen und/oder aliphatische Epoxyverbindungen verwendet werden.

7. Dentalmasse nach Anspruch 5 oder 6, **dadurch gekennzeichnet, dass** als Bestandteil (A) cycloaliphatische Epoxyverbindungen mit mindestens zwei Epoxygruppen oder cycloaliphatische Epoxyverbindungen mit mindestens vier Epoxygruppen oder cycloaliphatische Epoxyverbindungen mit zwei Epoxygruppen und cycloaliphatische Epoxyverbindungen mit mindestens vier Epoxygruppen verwendet werden.

8. Dentalmasse nach einem der Ansprüche 5 bis 7, **dadurch gekennzeichnet, dass** der Anteil der Epoxyverbindungen mit mindestens vier Epoxygruppen In den angemischten Zubereitungen 5 bis 60 Gew.-%, vorzugsweise 5 bis 50 Gew.-% beträgt.

9. Dentalmasse nach einem der Ansprüche 5 bis 8, **dadurch gekennzeichnet, dass** Verbindungen enthalten sind, die zur Bildung von Brönstedsäuren geeignet sind, und dass diese in einer Menge vorliegen, dass die Konzentration der erzeugten Brönstedsäure und der gegebenenfalls in freier Form eingesetzten Brönstedsäure 0,1 bis 5 Gew.-%, bezogen auf die angemischte Dentalmasse, beträgt.

10. Dentalmasse nach Anspruch 9, **dadurch gekennzeichnet, dass** die Brönstedsäuren durch Redoxreaktion aus Bisaryliodoniumsalzen erzeugt werden, und dass zusätzlich freie Brönstedsäuren vorliegen.

11. Dentalmasse nach Anspruch 10, **dadurch gekennzeichnet, dass** die gegebenenfalls zusätzlich vorliegenden freien Brönstedsäuren und die durch Redoxreaktionen aus Bisaryliodoniumsalzen erzeugten Brönstedsäuren identisch sind, oder dass die gegebenenfalis zusätzlich vorliegenden freien Brönstedsäuren und die durch Redoxreaktionen aus Bisaryliodoniumsalzen erzeugten Brönstedsäuren unterschiedlich sind.

12. Dentalmasse nach einem der Ansprüche 5 bis 11, **dadurch gekennzeichnet, dass** die brönstedsauren oder lewissauren Carbeniumlonen durch Reaktion von Alkyl- oder Aryl- oder gemischten Alkylarylhalogeniden mit Metallsalzen nach Mischung der beiden Komponenten erzeugt werden.

13. Dentalmasse nach Anspruch 12, **dadurch gekennzeichnet, daß** als Metallsalze Zink-, Eisen- und/oder Kupfersalze eingesetzt werden.

14. Dentalmasse nach einem der Ansprüche 5 bis 13, **dadurch gekennzeichnet, dass** das Anion der Brönstedsäuren auswählt ist aus der Gruppe, bestehend aus Hexafluorophosphat, Hexafluoroarsenat, Hexafluoroantimonat, Tetrakis(pentafluorophenyl)borat und Tetrafluoroborat.

15. Dentalmasse nach einem der Ansprüche 5 bis 13, **dadurch gekennzeichnet, dass** das Verhältnis zwischen der keine Epoxyverbindungen enthaltenden Komponente und der Epoxyverbindungen enthaltenden Komponente 1 : 10 bis 1 : 1 und vorzugsweise 1 : 5 bis 1 : 2 beträgt.

16. Verfahren zur Anmischung der Dentalmasse nach einem der Ansprüche 5 bis 15, **dadurch gekennzeichnet, dass** die Mischung der beiden Komponenten durch dynamische Mischverfahren oder mit Hilfe eines statischen Mischers erfolgt, der auf eine Doppelkammerkartusche aufgesetzt wird.

17. Verwendung der Zubereitungen nach einem der Ansprüche 1 bis 4 zur Herstellung von Dentalmassen, insbesondere zur Herstellung von Materialien für die Herstellung von zahntechnischen Modellen, Füllungen, provisorischen Kronen und Brücken sowie von Zementen.

18. Verwendung von Epoxyverbindungen enthaltenden Zusammensetzungen, die durch kationische Polymerisation, initiiert durch Lewis- und/oder Brönstedsäuren, aushärten, für dentale Zwecke, wobei die Lewis- und/oder Brönstedsäuren in Form von Vorläuferverbindungen eingesetzt werden, die zur Bildung von Lewis- und/oder Brönstedsäuren geeignet sind und bei oder nach dem Mischen der beiden Komponenten entweder durch chemische Umsetzung mittels Redoxreaktionen unter Verwendung von Bisaryliodoniumsalzen, Reduktionsmitteln und Kupferkomplexen in Verbindung mit Lewis- und/oder Brönstedsäuren in freier Form oder mittels durch Metallsalze unterstützter Dehalogenierung von Alkyl- halogeniden gebildet werden.

19. Verwendung von Epoxyverbindungen enthaltenden Zusammensetzungen nach Anspruch 18 zur Herstellung von zahntechnischen Modellen, Füllungen, provisorischen Kronen und Brücken sowie von Befestigungszementen.

## Claims

1. Two-component preparation, comprising the components (I) and (II), where at least one component contains epoxy compounds and the preparation, after the mixing of the two components, cure by cationic polymerization, initiated by Lewis and/or Brönsted acids, the Lewis and/or Brönsted acids being contained in the form of precursor compounds which are suitable for the formation of Lewis and/or Brönsted acids and are formed during or after the mixing of the two components either by chemical reaction by means of redox reactions using bisaryliodonium salts, reducing agents and copper complexes in combination with Lewis and/or Brönsted acids in free form or by means of dehalogenation of alkyl halides assisted by metal salts.

2. Preparation according to Claim 1, **characterized in that** the epoxy compounds and the compounds capable of the formation of Lewis and/or Brönsted acids, which do not react with the epoxy compounds, are present in any desired distribution in component (I) and/or in component (II).

3. Preparation according to Claim 1, **characterized in that**, in the case of a chemical reaction by means of dehalogenation of alkyl halides assisted by metal salts, it contains Lewis and/or Brönsted acids in free form as part of that component which contains no epoxy compounds.

4. Preparation according to Claim 3, **characterized in that** the epoxy compounds in component (I), the Lewis and/or Brönsted acids in free form in component (II) and the compounds which are suitable for the formation of Lewis and/or Brönsted acids, which do not react with the epoxy compounds, are present in any desired distribution in component (I) and/or (II).

5. Dental substance comprising preparations according to one of Claims 1 to 4, **characterized in that** it contains, divided over the components (I) and (II), the constituents:
(A) 10 to 80% by weight of epoxy compounds,
(B) 0.01 to 20% by weight of compounds which are capable of the formation of Lewis and/or Brönsted acids, and, if appropriate, free Lewis and/or Brönsted acids,
(C) 10 to 89.99% by weight of diluents,
(D) 0 to 79.99% by weight of modifiers,
the statement of the % by weight relating to the total mass of the mixed preparations.

6. Dental substance according to Claim 5, **characterized in that**, as constituent (A), cycloaliphatic epoxy compounds and/or aromatic epoxy compounds and/or aliphatic epoxy compounds are used.

7. Dental substance according to Claim 5 or 6, **characterized in that**, as constituent (A), cycloaliphatic epoxy compounds having at least two epoxy groups or cycloaliphatic epoxy compounds having at least four epoxy groups or cycloaliphatic epoxy compounds having two epoxy groups and cycloaliphatic epoxy compounds having at least four epoxy groups are used.

8. Dental substance according to one of Claims 5 to 7, **characterized in that** the proportion of the epoxy compounds having at least four epoxy groups in the mixed preparations is 5 to 60% by weight, preferably 5 to 50% by weight.

9. Dental substance according to one of Claims 5 to 8, **characterized in that** compounds are contained which are suitable for the formation of Brönsted acids, and that these are present in an amount such that the concentration of the Brönsted acid produced and of the Brönsted acid optionally employed in free form is 0.1 to 5% by weight, based on the mixed dental substance.

10. Dental substance according to Claim 9, **characterized in that** the Brönsted acids are produced by redox reactions from bisaryliodonium salts, and **in that** free Brönsted acids are additionally present.

11. Dental substance according to Claim 10, **characterized in that** the optionally additionally present free Brönsted acids and the Brönsted acids produced by redox reactions from bisaryliodonium salts are identical, or **in that** the optionally additionally present free Brönsted acids and the Brönsted acids produced by redox reactions from bisaryliodonium salts are different.

12. Dental substance according to one of Claims 5 to 11, **characterized in that** the Brönsted acid or Lewis acid carbenium ions are produced by reaction of alkyl halides or aryl halides or mixed alkyl-aryl halides with metal salts after mixing of the two components.

13. Dental substance according to Claim 12, **characterized in that** the metal salts employed are zinc, iron and/or copper salts.

14. Dental substance according to one of Claims 5 to 13, **characterized in that** the anion of the Brönsted acids is selected from the group consisting of hexafluorophosphate, hexafluoro-arsenate, hexafluoroantimonate, tetrakis(penta-fluorophenyl)borate and tetrafluoroborate.

15. Dental substance according to one of Claims 5 to 13, **characterized in that** the ratio between the component containing no epoxy compounds and the component containing epoxy compounds is 1:10 to 1:1 and preferably 1:5 to 1:2.

16. Process for the mixing of the dental substance according to one of Claims 5 to 15, **characterized in that** the mixing of the two components is carried out by means of a dynamic mixing process or with the aid of a static mixer which is attached to a double-chamber cartridge.

17. Use of the preparations according to one of Claims 1 to 4 for the production of dental substances, in particular for the production of materials for the production of dental models, fillings, temporary crowns and bridges, and of cements.

18. Use of compositions containing epoxy compounds which cure by cationic polymerization, initiated by Lewis and/or Brönsted acids, for dental purposes, the Lewis and/or Brönsted acids being employed in the form of precursor compounds which are suitable for the formation of Lewis and/or Brönsted acids and are formed during or after the mixing of the two components either by chemical reaction by means of redox reactions using bisaryliodonium salts, reducing agents and copper complexes in combination with Lewis and/or Brönsted acids in free form or by means of dehalogenation of alkyl halides assisted by metal salts.

19. Use of compositions containing epoxy compounds according to Claim 18 for the production of dental models, fillings, temporary crowns and bridges, and of fixative cements.

## Revendications

1. Composition à deux composants, comprenant les composants (I) et (II), au moins un composant contenant des composés époxy et la composition durcissant après le mélange des deux composants par polymérisation cationique, initiée par des acides de Lewis et/ou de Brönsted, les acides de Lewis et/ou de Brönsted étant contenus sous forme de composés précurseurs, qui conviennent pour la formation d'acides de Lewis et/ou de Brönsted et qui sont formés lors du mélange des deux composants ou après celui-ci, par transformation chimique au moyen de réactions redox avec utilisation de sels de bisaryliodonium, de réducteurs et de complexes de cuivre en association avec des acides de Lewis et/ou de Brönsted sous forme libre ou par déshalogénation d'halogénures d'alkyle soutenue par des sels métalliques.

2. Composition selon la revendication 1, **caractérisée en ce que** les composés époxy et les composés qui sont aptes à former des acides de Lewis et/ou de Brönsted, qui ne réagissent pas avec les composés époxy, peuvent se trouver dans une répartition quelconque dans les composants (I) et/ou les composant (II).

3. Composition selon la revendication 1, **caractérisée en ce qu'**elle contient, dans le cas d'une transformation chimique au moyen d'une déshalogénation soutenue par des sels métalliques d'halogénures d'alkyle, en plus, comme partie du composant qui ne présente pas de composés époxy, des acides de Lewis et/ou de Brönsted sous forme libre.

4. Composition selon la revendication 3, **caractérisée en ce que** les composés époxy se trouvent dans le composant (I), les acides de Lewis et/ou de Brönsted sous forme libre se trouvent dans le composant (II) et les composés qui sont aptes à former des acides de Lewis et/ou de Brönsted et qui ne réagissent pas avec les composés époxy se trouvent dans une répartition quelconque dans le composant (I) et/ou le composant (II).

5. Masses dentaires réalisées à partir des compositions selon l'une quelconque des revendications 1 à 4, **caractérisées en ce qu'**elles contiennent, répartis sur les composants (I) et (II), les constituants :
(A) 10 à 80% en poids de composés époxy,
(B) 0,01 à 20% en poids de composés aptes à la formation d'acides de Lewis ou de Brönsted et le cas échéant d'acides de Lewis et/ou de Brönsted libres,
(C) 10 à 89,99% en poids de diluants,
(D) 0 à 79,99% en poids d'agents de modification
les indications des% en poids se rapportant à la masse totale des compositions mélangées.

6. Masses dentaires selon la revendication 5, **caractérisées en ce qu'**on utilise comme constituant (A) des composés époxy cycloaliphatiques et/ou des composés époxy aromatiques et/ou des composés époxy aliphatiques.

7. Masses dentaires selon la revendication 5 ou 6, **caractérisées en ce qu'**on utilise comme constituant (A) des composés époxy cycloaliphatiques comprenant au moins deux groupes époxy ou des composés époxy cycloaliphatiques comprenant au moins quatre groupes époxy ou des composés époxy cycloaliphatiques comprenant deux groupes époxy et des composés époxy cycloaliphatiques comprenant au moins quatre groupes époxy.

8. Masses dentaires selon l'une quelconque des revendications 5 à 7, **caractérisées en ce que** la proportion des composés époxy présentant au moins quatre groupes époxy dans les compositions mélangées est de 5 à 60% en poids, de préférence de 5 à 50% en poids.

9. Masses dentaires selon l'une quelconque des revendications 5 à 8, **caractérisées en ce que** des composés qui conviennent pour la formation d'acides de Brönsted sont contenus et **en ce qu'**ils sont présents en une quantité telle que la concentration obtenue en acides de Brönsted produits et en acides de Brönsted le cas échéant utilisés sous forme libre est de 0,1 à 5% en poids, par rapport à la masse dentaire mélangée.

10. Masses dentaires selon la revendication 9, **caractérisées en ce que** les acides de Brönsted sont produits par une réaction redox à partir de sels de bisaryliodonium et **en ce que** des acides de Brönsted libres supplémentaires sont présents.

11. Masses dentaires selon la revendication 10, **caractérisées en ce que** les acides de Brönsted libres le cas échéant présents en supplément et les acides de Brônsted produits par les réactions redox à partir de sels de bisaryliodonium sont identiques ou **en ce que** les acides de Brönsted libres le cas échéant présents en supplément et les acides de Brönsted produits par des réactions redox à partir de sels de bisaryliodonium sont différents.

12. Masses dentaires selon l'une quelconque des revendications 5 à 11, **caractérisées en ce que** les ions carbénium acides de Brönsted ou acides de Lewis sont produits par réaction d'halogénures d'alkyle, d'aryle ou d'alkylaryle mixtes avec des sels métalliques après mélange des deux composants.

13. Masses dentaires selon la revendication 12, **caractérisées en ce qu'**on utilise comme sels métalliques des sels de zinc, de fer et/ou de cuivre.

14. Masses dentaires selon l'une quelconque des revendications 5 à 13, **caractérisées en ce que** l'anion des acides de Brönsted est choisi dans le groupe constitué par l'hexafluorophosphate, l'hexafluoroarsénate, l'hexafluoroantimonate, le tétrakis(pentafluorophényl)borate et le tétrafluoroborate.

15. Masses dentaires selon l'une quelconque des revendications 5 à 13, **caractérisées en ce que** le rapport entre le composant ne contenant pas de composés époxy et le composant contenant des composés époxy est de 1 : 10 à 1 : 1 et de préférence de 1 : 5 à 1 : 2.

16. Procédé pour le mélange des masses dentaires selon l'une quelconque des revendications 5 à 15, **caractérisé en ce que** le mélange des deux composants est réalisé par des procédés de mélange dynamiques ou à l'aide d'un mélangeur statique qui est placé sur la cartouche à deux chambres.

17. Utilisation des compositions selon l'une quelconque des revendications 1 à 4 pour la préparation de masses dentaires, en particulier pour la préparation de matériaux pour la réalisation de moules techniques dentaires, de remplissages, de couronnes et de bridges provisoires ainsi que de ciments.

18. Utilisation de compositions contenant des composés époxy, qui durcissent par polymérisation cationique, initiée par des acides de Lewis et/ou de Brönsted, à des fins dentaires, les acides de Lewis et/ou de Brönsted étant utilisés sous forme de composés précurseurs, qui conviennent pour la formation d'acides de Lewis et/ou de Brönsted et qui sont formés lors du mélange des deux composants ou après celui-ci, par transformation chimique au moyen de réactions redox avec utilisation de sels de bisaryliodonium, de réducteurs et de complexes de cuivre en association avec des acides de Lewis et/ou de Brönsted sous forme libre ou par déshalogénation d'halogénures d'alkyle soutenue par des sels métalliques.

19. Utilisation de compositions contenant des composés époxy selon la revendication 18 pour la réalisation de moules techniques dentaires, de remplissages, de couronnes et de bridges provisoires ainsi que de ciments de fixation.
